# EUROPEAN PATENT SPECIFICATION

(11) **EP 1 438 435 B1**
(45) Date of publication and mention of the grant of the patent: **31.10.2007**
(21) Application number: 02797818.8
(22) Date of filing: 03.09.2002
(51) Int. Cl.: C12Q 1/68

(54) **Methods for blocking nonspecific hybridisations of nucleic acid sequences**
Verfahren zur Blockierung von unspezifischen Hybridisierungen von Nukleinsäuresequenzen
Procédés de blocage de l'hybridation non spécifique des séquences nucléotidiques

(30) Priority: 31.08.2001 US 316116 P
(43) Date of publication of application: 21.07.2004
(73) Proprietor: DATASCOPE INVESTMENT CORP., Montvale, N.J. 07645 (US)
(72) Inventor: KADUSHIN, James M., Datascope Investment Corp., Montvale, NJ 07645 (US); GETTS, Robert C., Datascope Investment Corp., Montvale, NJ 07645 (US); ZAK, Arieh S., Datascope Investment Corp., Montvale, NJ 07645 (US)
(74) Representative: Amery, Marcus James
(86) International application number: PCT/US2002/027799
(87) International publication number: WO 2003/020902

(56) References cited:
- WO-A-00/56920
- WO-A-01/66555
- WO-A-02/33125
- WO-A1-00/56748
- US-A- 6 046 038
- US-A- 6 077 673
- US-A- 6 110 674
- US-A- 6 130 065
- US-A- 6 165 750
- US-B1- 6 277 568
- US-B1- 6 387 624

### SEQUENCE LISTING

<110> DATASCOPE INVESTMENT CORP.
<120> Methods for Blocking Non-Specific Hybridizations of Nucleic Acid Sequences
<130> P 001 822 EP; 4081.008.075 (EPO)
<140> EP Appl. 02 797 818.8 (PCT/US02/27799)
   <141> 2002-09-03
<150> 60/316,116
   <151> 2001-08-31
<160> 4
<170> PatentIn version 3.3
<210> 1
   <211> 28
   <212> DNA
   <213> Artificial Sequence
<220>
   <223> RT primer capture sequence
<220>
   <221> misc_feature
   <222> (1)..(28)
<400> 1
   ggcctcactg cgcgtcttct gtcccgcc 28
<210> 2
   <211> 31
   <212> DNA
   <213> Artificial Sequence
<220>
   <223> RT primer capture sequence
<220>
   <221> misc_feature
   <222> (1)..(31)
<400> 2
   cctgttgctc tatttcccgt gccgctccgg t 31
<210> 3
   <211> 12
   <212> RNA
   <213> Eukaryotic Poly A tail
<220>
   <221> polyA_site
   <222> (1)..(12)
<400> 3
   aaaaaaaaaa aa 12
<210> 4
   <211> 12
   <212> DNA
   <213> Artificial Sequence
<220>
   <223> Poly T for hybridization to Poly A tail
<220>
   <221> misc_feature
   <222> (1)..(12)
<400> 4
   tttttttttt tt 12

## Description

### Field of the Invention

The present invention relates to DNA microarrays, more particularly to methods for blocking non-specific interactions during the hybridization of nucleic acid sequence samples to a microarray.

### Background of the Invention

Changes in gene expression patterns or in a DNA sequence can have profound effects on biological functions. Such variations in gene expression may result in altered physiologic and pathologic processes. Developing DNA technologies are providing rapid and cost-effective methods for identifying gene expression and genetic variations on a large-scale level.

One high-speed technology useful for DNA analysis is the DNA microarray which includes a plurality of distinct DNA or gene probes (i.e., polynucleotides) distributed spatially, and stably associated with a substantially planar substrate such as a plate of glass, silicon or nylon membrane. Such microarrays have been developed and are used in a range of applications such as analyzing a sample for the presence of gene variations or mutations (i.e. genotyping), or for patterns of gene expression, while performing the equivalent of thousands of individual "test-tube" experiments carried out in a short period of time.

All microarrays operate on a similar principle: a substantially planar substrate such as a glass coverslide is coated with a grid of tiny spots of about 20 to 100 microns in diameter; each spot (i.e. feature) contains millions of copies of a short sequence of DNA or nucleotides; and a computer keeps track of each sequence at a predetermined feature. To make an analysis, messenger RNA (mRNA) is extracted from a sample of cells. Using enzymes, millions of copies of the mRNA molecules are reproduced. Copies of complementary DNA (cDNA) are generated from the mRNA through reverse transcription. The cDNA copies are tagged with a marker or label such as a fluorescent marker and broken up into short fragments. The tagged fragments are washed over the microarray and left overnight, to allow the tagged fragments to hybridize with the DNA attached to the microarray.

After hybridization, the features on the microarray that have paired with the fluorescent cDNA emit a fluorescent signal that can be viewed with a microscope or detected by a computer. In this manner, one can learn which sequences on the microarray match the cDNA of the test sample. Although there are occasional mismatches, the employment of millions of probes in each spot or feature ensure fluorescence is detected only if the complementary cDNA is present. The more intense the fluorescent signal, (i.e. the brighter the spot) the more matching cDNA was present in the cell.

One area in which the microarrays are useful is in gene expression analysis. In gene expression analysis utilizing microarrays, an array of "probe" oligonucleotides is contacted with a nucleic acid sample of interest, i.e. target, such as cDNA generated from mRNA extracted from a particular tissue type. Contact is carried out under hybridization conditions and unbound nucleic acid is then removed. The resultant pattern of hybridized nucleic acid provides information regarding the genetic profile of the sample tested. Genetic profile is meant to include information regarding the types of nucleic acids present in the sample, (e.g. the types of genes to which they are complementary, as well as the copy number of each particular nucleic acid in the sample). Gene expression analysis may be use in a variety of applications, including, for example, the identification of novel expression of genes, the correlation of gene expression to a particular phenotype, screening for disease predisposition, and identifying the effect of a particular agent on cellular gene expression, such as in toxicity testing.

Using known methods, a plurality of gene probes are affixed or printed on the surface of a microarray such as by robotic or laser lithographic processes. Labelled target molecules are subsequently applied to those probes, and the array is washed to remove target molecules that have not hybridized.

For example, a sample for use on a microarray can be prepared using messenger RNA (mRNA) or total RNA extracted from a sample of cells. The mRNA serving as a template, is reverse transcribed to yield complementary DNA (cDNA) target molecules. One or more labels or markers such as fluorescence are directly incorporated into the copies of cDNA during the reverse transcription process (the labelling being conducted to allow subsequent detection of these cDNA molecules by scanning for their fluorescent signal). The labeled copies of cDNA are broken up into short fragments and washed over the microarray. Under suitable hybridization conditions, the labeled fragments are hybridized or coupled with complementary nucleic acid sequences (i.e. gene probes) attached to the features of the microarray for ready detection thereof. This labeling method has been commonly referred to as "direct incorporation".

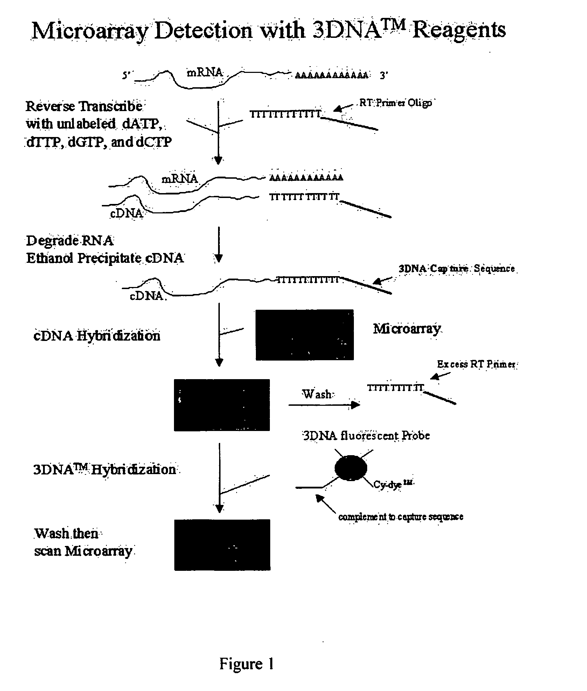

In an alternate method (as shown in Figure 1), the complementary DNA (cDNA) is prepared from a mRNA sample comprised of total RNA or poly(A)⁺ RNA, along with a large quantity of nucleotide bases (deoxynucleotide triphosphate, DNTP), enzymes and reverse transcription (RT) primer oligonucleotides with capture sequence portions appended thereto. Alternatively, any other processes for synthesizing complementary deoxyribonucleic acid (cDNA) from ribonucleic acid (RNA) can be used as well, including, but not limited to the methods of Von Gelder, Von Zastrow, Barchas and Eberwine disclosed in U.S. Patent Nos. 5,716,787 and 5,891,636.

The newly formed cDNA is then isolated from the mRNA sample and precipitated with ethanol. The cDNA is then suspended in a cDNA hybridization buffer for hybridizing the cDNA to the microarray with the complementary gene probes and incubated overnight. Following hybridization of the cDNA to the prepared microarray, the microarray is washed to remove any excess RT primer oligonucleotide. The cDNAs are labelled using molecules (e.g. dendrimers) having both a label and a sequence complementary to the capture sequence.

Whether the cDNA is prepared using direct incorporation or a capture sequence, upon hybridization of the cDNA to the microarray, a detectable signal (e.g. fluorescence) is emitted for a positive outcome from each feature containing a cDNA fragment hybridized with a complementary gene probe attached thereto. The detectable signal is visible to an appropriate sensor device or microscope, and may then be detected by the computer or user to generate a hybridization pattern. Since the nucleic acid sequence at each feature is known, any positive outcome (i.e. signal generation) at a particular feature indicates the presence of the complementary cDNA sequence in the sample cell. Although there are occasional mismatches, the attachment of millions of gene probes at each spot or feature ensures that the detectable signal is strongly emitted only if the complementary cDNA of the test sample is present.

WO 01/66555A teaches a method for assay and detection of nucleic acids which uses a decreased number of steps to reduce the time and effort typically required for assaying a sample on a microarray. WO 01/66555A, however, does not teach the use of an LNA reagent to block unspecific hybridization on a microarray.

WO 02/33125A1 teaches a method for detecting nucleic acids by incorporating complementary labeled dendrimeric molecules into a target or probe nucleic acid strand. WO 02/33125A1, however, does not teach the use of an LNA reagent to block unspecific hybridization on a microarray.

WO 00/56920 teaches a method for use of LNA which is substantially complementary to a target nucleic acid for hybridization of the LNA to that target nucleic acid. WO 00/56920, however, does not teach the use of an LNA reagent to block unspecific hybridization on a microarray.

In conjunction with the present inventions, dendritic nucleic acid molecules are particularly preferred for their detection capabilities (although any type of labelled molecules can be utilized with the inventions disclosed herein). Dendritic nucleic acid molecules, or dendrimers are complex, highly branched molecules, comprised of a plurality of interconnected natural or synthetic monomeric subunits of double-stranded DNA. Dendrimers are described in greater detail in Nilsen et al., Dendritic Nucleic Acid Structures, J. Theor. Biol., 187, 273-284 (1997); in Stears et al., A Novel, Sensitive Detection System for High-Density Microarrays Using Dendrimer Technology, Physiol. Genomics, 3: 93-99 (2000); and in various U.S. patents, such as U.S. Patent Nos. 5,175,270; 5,484,904; 5,487,973; 6,072,043; 6,110,687; and 6,117,631.

Dendrimers comprise two types of single-stranded hybridization "arms" on the surface which are used to attach two key functionalities. A single dendrimer molecule may have at least one hundred arms of each type on the surface. One type of arm is used for attachment of a specific targeting molecule to establish target specificity and the other is used for attachment of a label or marker. The molecules that determine the target and labeling specificities of the dendrimer are attached either as oligonucleotides or as oligonucleotide conjugates. Using simple DNA labeling, hybridization, and ligation reactions, a dendrimer molecule may be configured to act as a highly labeled, target specific probe.

The prepared mixture is formulated in the presence of a suitable buffer to yield a dendrimer hybridization mixture containing dendrimers with fluorescent labels attached to one type of "arm", and with oligonucleotides attached to another type of "arm", complementary to the capture sequences of the RT primer bound cDNA fragments. An oligonucleotide designed to block non-specific interaction of the cDNA or the dendrimer to the nucleic acid spotted on the array surface is also added at this time; blocking oligonucleotides containing the multiplicities of the same nucleic acid base may be used for blocking long stretches of the same complementary base found on the cDNA derived from the RNA sample and the nucleic acid probes on the microarray surface. In the present invention, as disclosed below, blocking oligonucleotides having a modified nucleotide which results in a change of melting temperature (Tm) have been found to be superior to prior blocking molecules.

The dendrimer hybridization mixture containing the dendrimer molecules is then added to the microarray and incubated overnight to generate a hybridization pattern. Subsequent to the dendrimer-to-cDNA hybridization, the microarray is washed to purge any excess unhybridized dendrimers. The microarray is scanned to detect the signal generated by the label to enable gene expression analysis of the hybridization pattern. One of the drawbacks using this method includes the undue time and labor required to prepare the sample and to perform the assay including the hybridization and washing steps.

It would be highly desirable to significantly reduce the amount of time and labor expended in preparation of the sample and performing the assay without sacrificing desirable attributes such as sensitivity, low background "noise", and minimal "false positives". It would be a significant advance in the art of gene expression detection microarrays to further provide a method which significantly reduces the complexity and the steps needed to prepare gene samples and the assay for gene expression analysis, and which can be carried out using conventional laboratory reagents, equipment and techniques. Accordingly, it is an object of the present invention to achieve such objectives using methods which block non-specific hybridizations on the array.

### Summary of the Invention

The present invention relates generally to methods for blocking non-specific hybridization between nucleic acid sequences. In accordance with the invention, a method is provided comprising the step of using a blocking reagent to minimize or eliminate non-specific interactions during the hybridization of nucleic acid molecules, wherein the blocking reagent has at least one modified nucleotide therein. This modified nucleotide is a nucleotide which is a modified version of a standard DNA or RNA nucleotide, while yet still obeying Watson-Crick base pairing rules. Thus, modified nucleotides have an affinity for the complementary standard DNA and RNA nucleotide, but that affinity is different from that of standard DNA nucleotides for their complements. As a result, the nucleic acid sequences incorporating them have a higher or lower melting temperature (Tm) than a standard double stranded sequence that only incorporates standard DNA or RNA nucleotides therein. For example, a modified nucleotide adenine base (A') can be provided having an affinity for a standard thymine base (T) which is greater than the affinity of a standard adenine base (A) for that same standard thymine base (T), and so forth. As a result, and as a further advantage of the present invention, the blocking reagent allows an applied stringency, i.e. the ability to vary the discrimination between specific and non-specific binding interactions by varying a physical parameter.

The method of the present invention may be used in a range of applications, and is particularly advantageous for use in association with microarrays. For example, the present invention can be used to provide significant reduction in the amount of non-specific hybridization and resulting signal from the hybridization of cDNA and other nucleic acid samples to complementary probes on the microarray surface. It thus results in a microarray assay with excellent sensitivity and low background "noise" and minimal "false positives". The parameters of the assay can be adjusted to result in the desired degree of efficiency and specificity for the desired hybridization of labelled target molecule to probe molecules on the array by adjusting the physical parameters of the reaction conditions, such as the reaction temperature or the number of modified nucleotides in the blocking reagent.

The blocking reagent itself is a molecule that will bind to a nucleic acid sequence, to prevent another nucleic acid from binding thereto, particularly, sequences that would bind non-specifically. It is preferably a molecule which is a nucleic acid sequence in its entirety, but can alternately be a molecule which incorporates a nucleic acid sequence therein.

One of ordinary skill in the art can choose or design the sequence of the blocking reagent based on the expected nucleotide sequences in the application that could result in non-specific binding, and can likewise choose which types of modified nucleotides to use, based on the expected conditions.

In the case of a microarray, for example, the blocking reagent is used to bind to probe sequences that are known to cause non-specific binding by labelled target molecules, with certain such probe sequences being well known in the art. For example, the procedures commonly used for generating arrays result in nucleotide sequences at each spot that have stretches of poly-A on the array. Ordinarily, the poly-T tail of a labelled cDNA target molecule would potentially hybridize to those poly-A sequences, resulting in non-specific signal, in place of hybridization of the cDNA sequence to any complementary genes of probe molecules on the array. As a result, to eliminate non-specific signal in this case, one designs a reagent to block non-specific hybridization to the poly-T sequences by using a blocking reagent in the form of a poly-A oligonucleotide having modified nucleotides incorporated therein. These poly-A oligonucleotides bind tightly to the poly-T sequences preventing any binding of the cDNA poly-T sequences to the array which would result in non-specific signal. In an analogous fashion, stretches of poly-T on the array can be blocked from binding to the poly-A of an target RNA molecules by using a poly-A blocking reagent. Thus, in the most common applications of the present invention, the blocking reagent consists of a poly- T or poly-A sequence. However, while poly-A and poly-T hybridizations are the most common form of non-specific binding, the present invention is not limited to use with those sequences. Rather, the blocking reagent can be provided with a complementary sequence to any probe or target sequence that could result in undesirable non-specific binding interactions.

In the present invention the modified nucleotide(s) of the blocking reagent are Locked Nucleic Acid nucleotides ("LNA" - modified bicyclic monomeric units with a 2'-O - 4'-C methylene bridge). Such LNA nucleotides have a stronger affinity for the probe nucleotides than DNA or RNA nucleotides, such that hybridization of the blocking reagent results in a structure which has a higher melting temperature than a traditional double stranded DNA sequence. Alternatively or additionally, the blocking reagent includes Peptide Nucleic Acids ("PNA) therein (i.e. modified nucleotide bases having a peptide backbone). However, other modified nucleotides can be used consistent with the invention as well.

### Brief Description of the Drawings

The following drawings in which like reference characters indicate like parts are illustrative of embodiments of the invention and are not to be construed as limiting the invention as encompassed by the claims forming part of the application.
Figure 1 is a schematic representation of one of the methods of the prior art for preparing a microarray for detection and assay of a nucleic acid sequence sample;
Figure 2 is a schematic representation of a method for preparing a microarray for detection and assay of a nucleic acid sequence sample in one embodiment of the present invention;
Figure 3 is a schematic representation of a method for preparing a microarray for detection and assay of a nucleic acid sequence sample in another embodiment of the present invention; and
Figure 4 shows the structure of LNA in comparison to DNA and RNA.
Figure 5 shows the structure of PNA in comparison to DNA.

### Detailed Description of the Invention and the Preferred Embodiments

Before the present invention is further described, it is to be understood that the invention is not limited to the particular embodiments of the invention described herein, as variations of the particular embodiments may be made and still fall within the scope of the invention or the appended claims. It is also to be understood that the terminology employed is for the purpose of describing particular embodiments, and is not intended to be limiting.

The present invention is generally directed to a method for detection and assay on a microarray in a manner that provides a significant reduction in non-specific signal. As is known in the art, non-specific signal relates to binding of the sample to molecules on the array surface which does not provide useful or relevant information regarding the identity of the sample (the target). For example, when the target molecules are cDNA prepared from a sample having mRNA with a poly-A tail, a poly-T tail is present which results from the presence of creating the cDNA, and which can result in target to probe binding which does not provide any useful or relevant information regarding the sequence of the mRNA that the cDNA was derived from.

The method of the present invention provides the advantage of low background "noise", and minimal "false positives" required for laboratory and clinical use. The cost effective and efficient manner by which the nucleic acid sequence samples are prepared and by which the method of the present invention can be implemented using conventional laboratory techniques, equipment and reagents, makes them especially suitable for research and clinical use.

To achieve these objectives, the present invention utilizes blocking reagents which reduce nonspecific hybridization of nucleic acid molecules to other nucleic acid sequences. These blocking reagents are reagents having nucleic acid sequences reagents with modified nucleotides therein. In the invention, the blocking reagents have one or more LNA (Locked Nucleic Acid) residues as the modified nucleotide(s) in the nucleic acid sequence. In alternate embodiments, one or more PNA residues or other modified nucleotides can be used.

LNA molecules are novel DNA analogues that obey Watson-Crick base pairing rules, and form DNA or RNA-heteroduplexes with high thermal stability. In the past, for example, they have been shown to have an excellent ability to discriminate between matching and mismatching target sequences in Single Nucleotide Polymorphism detection.

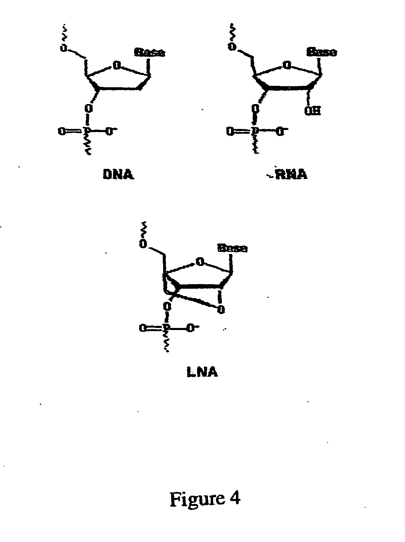

As shown in Figure 4, the normal conformational freedom of the furanose ring has been restricted in these molecules using a methylene linker connecting the 2' - O position to the 4' - C position. Preferably, the LNA molecules are obtained from Proligo, LLC of Boulder, Colorado (www. proligo.com), or from Exiqon A/S of Vedbaek, Denmark (www. exiqon.com). Alternately, they can be synthesized using standard phosphoramidite chemistry using DNA-synthesizers. (See also, Sanjay Singh et al., "LNA (Locked Nucleic Acids): Synthesis and High Affinity Nucleic Acid Recognition", Chemical Communications, Royal Society of Chemistry, GB, No. 4, Feb. 21, 1998, which is fully incorporated herein by reference.) As an alternative to the structure shown in Figure 4, other analogues can also be used wherein the 2'-oxy atom is replaced by either nitrogen or sulfur. In further alternate embodiments, the a-L-ribo diastereoisomeric form of LNA can be used.

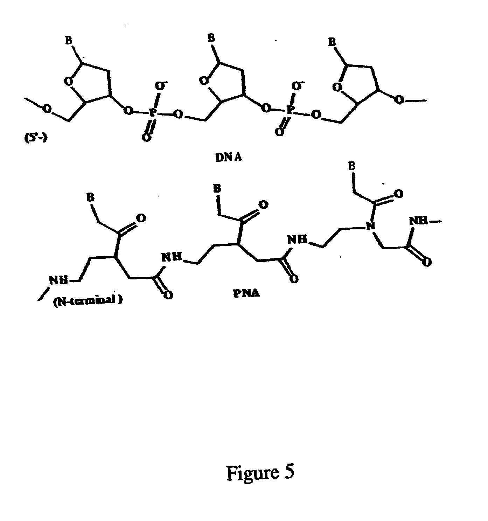

With respect to PNA, the PNA monomer is 2-aminoethyl glycine linked by a methylenecarbonyl linkage to one of the four bases (adenine, guanine, thymine, or cytosine) found in DNA. Unlike standard nucleotides, PNA's lack pentose sugar phosphate groups. The general structure of PNA is shown in Figure 5.

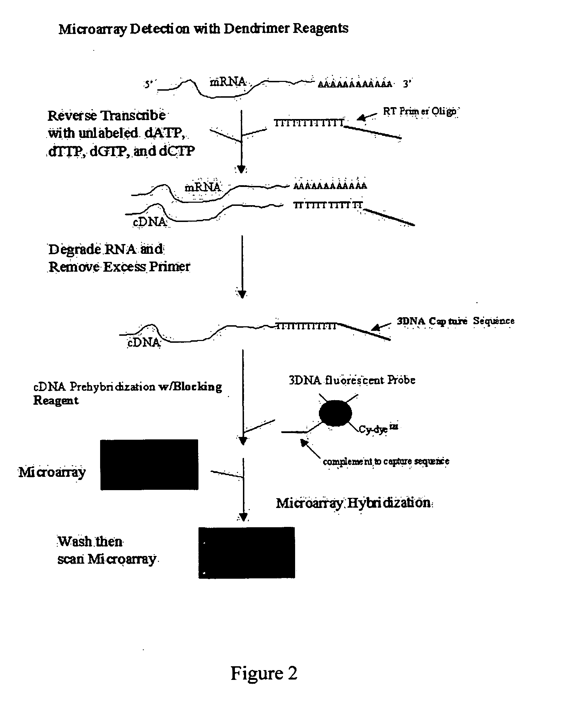

Like amino acids, PNA monomers have amino and carboxyl termini. The PNA monomers are linked by peptide bonds into a single chain oligomer. By convention, the PNA oligomer is depicted like a peptide with its N-terminus at the first position (Fig. 2). This end corresponds to the 3' end of a DNA or RNA strand, with the the N-terminus of a PNA hybridizing to the 5'-end of complementary single-stranded DNA. Thus, unlike the 5' to 3' convention in writing nucleic acid sequences, PNA sequences are usually written from 3' to 5'. Further details regarding them are provided in B. Hyrup and P.E. Nielsen, Peptide Nucleic Acids (PNA): Synthesis, Properties and Potential Applications, Bioorganic and Medicinal Chemistry, vol. 4. no. 1. pp. 5-23 (Elsevier Science Ltd., Great Britain, 1996).

While PNA monomers can be used consistent with the invention, it can present certain drawbacks in some applications, due to such factors as the fact that it, currently, can only be synthesized into oligomers as long as 20 bases. Likewise, its its melting temperature tends to top out at certain maximum levels, as opposed to LNA which does not present either limitation. As a result, LNA is generally preferred as the modified nucleotide of choice for the present blockers.

In the methods of the present invention, an array of DNA or gene probes fixed or stably associated with the surface of a substantially planar substrate is contacted with a sample of target nucleic acids under hybridization conditions sufficient to produce a hybridization pattern of complementary probe/target complexes. A variety of different microarrays which may be used are known in the art. The hybridized samples of nucleic acids are then targeted by labeled probes and hybridized to produce a detectable signal corresponding to a particular hybridization pattern. The individual labeled probes hybridized to the target nucleic acids are all capable of generating the same signal of known intensity. Thus, each positive signal in the microarray can be "counted" in order to obtain quantitative information about the genetic profile of the target nucleic acid sample.

The DNA or gene probes of the microarrays which are capable of sequence specific hybridization with target nucleic acid may be polynucleotides or hybridizing analogues or mimetics thereof, including, but not limited to, nucleic acids in which the phosphodiester linkage has been replaced with a substitute linkage group, such as phophorothioate, methylimino, methylphosphonate, phosphoramidate, guanidine and the like, nucleic acids in which the ribose subunit has been substituted, e.g. hexose phosphodiester; peptide nucleic acids, and the like. The length of the probes will generally range from 10 to 1000 nucleotides. In some embodiments of the invention, the probes will be oligonucleotides having from 15 to 150 nucleotides and more usually from 15 to 100 nucleotides. In other embodiments the probes will be longer, usually ranging in length from 150 to 1000 nucleotides, where the polynucleotide probes may be single or double stranded, usually single stranded, and may be PCR fragments amplified from cDNA. The DNA or gene probes on the surface of the substrates will preferably correspond to known genes of the physiological source being analyzed and be positioned on the microarray at a known location so that positive hybridization events may be correlated to expression of a particular gene in the physiological source from which the target nucleic acid sample is derived. Because of the manner in which the target nucleic acid sample is generated, as described below, the microarrays of gene probes will generally have sequences that are complementary to the non-template strands of the gene to which they correspond.

The substrates with which the gene probes are stably associated may be fabricated from a variety of materials, including plastic, ceramic, metal, gel, membrane, glass, and the like. The microarrays may be produced according to any convenient and conventional methodology, such as preforming the gene probes and then stably associating them with the surface of the support or growing the gene probes directly on the support. A number of different microarray configurations and methods for their production are known to those of skill in the art, one of which is described in Science, 283, 83, 1999.

The term "label" is used herein to refer to agents that are capable of providing a detectable signal, either directly or through interaction with one or more additional members of a signal producing system. Labels that are directly detectable and may find use in the present invention include fluorescent labels such as fluorescein, rhodamine, BODIPY, cyanine dyes, fluorescent dye phosphoramidites, and the like; and radioactive isotopes, such as ³²S, ³²P, ³H, etc.; and the like. Examples of labels that provide a detectable signal through interaction with one or more additional members of a signal producing system include capture moieties that specifically bind to complementary binding pair members, where the complementary binding pair members comprise a directly detectable label moiety, such as a fluorescent moiety as described above. The label is one which preferably does not provide a variable signal, but instead provides a constant and reproducible signal over a given period of time.

In accordance with the method of the present invention, a desired microarray is provided having the probe nucleic acid sequences stably affixed thereto. In addition, a sample is provided having the target molecules of interest for study. The target molecules are labelled by any desired method, whether direct incorporation of label molecules or other methods such as hybridization of the target to a suitable label molecule such as a dendrimer, as discussed more fully below. The target molecules can be labelled prior to or after application of target to the array, although prior labelling is generally preferred.

Thus, in one preferred embodiment, a method is provided which comprises the steps, in any suitable order, of: using a microarray wherein the microarray comprises a plurality of features, each of the features having a probe nucleotide sequence; applying a sample to the microarray, wherein the sample comprises target molecules for binding to any of the probe nucleotide sequences on the microarray that are complementary to the target molecules; and using a blocking reagent to reduce non-specific binding between the target and the probe molecules, wherein the blocking reagent comprises a sequence of nucleic acids comprising at least one modified nucleotide, the modified nucleotide being free of a peptide backbone; wherein the blocking reagent comprising the modified nucleotide has a higer melting temperature (Tm) relative to a reagent with the same sequence of nucleic acids but having a standard nucleotide base in place of the modified nucleotide.

In a further preferred embodiment, a method is provided which comprises the steps, in any suitable order, of: using a microarray, the microarray having a plurality of features thereon, each of the features having a probe nucleotide sequence; applying a sample to the microarray, wherein the sample has target molecules therein for binding to any complementary probe sequences on the microarray; and using an LNA reagent as a blocking reagent to reduce non-specific binding between the target and probe molecules, wherein the LNA reagent is a DNA oligonucleotide containing residues of Locked Nucleic Acid, the Locked Nucleic Acid residues being modified bicyclic monomeric units with a 2'-O- 4'-C methylene bridge.

In a further preferred embodiment, the target molecules have a label or tag directly or indirectly incorporated therein. In a further preferred embodiment, the target molecules comprise a cDNA reagent obtained from mRNA of a target sample, although any target molecules can be used consistent with the invention.

Thus, the blocking reagent is used to bind nucleic acid sequences which could result in non-specific binding between the target molecules and the probe molecules. Usually, the blocking reagent is applied to the microarray to bind nucleic acid sequences of the probes, "blocking" the probe sequences from binding to the target. However, in an alternate embodiment, the blocking reagent can be applied to the sample to block the nucleic acid sequences of the target. With either embodiment, consistent with the invention, the blocking reagent can be applied prior to or concurrent with application of the sample to the array.

In further preferred embodiments of the invention, dendritic nucleic acid molecules or dendrimers are used as the label. Dendrimers are complex, highly branched molecules, comprised of a plurality of interconnected natural or synthetic monomeric subunits of double-stranded DNA. Dendrimers are described in Nilsen et al., Dendritic Nucleic Acid Structures, J. Theor. Biol., 187, 273-284 (1997). Further information regarding the structure and production of dendrimers is disclosed in U.S. Pat. Nos. 5,175,270, 5,484,904, and 5,487,973.

Dendrimers comprise two types of single-stranded hybridization "arms" on the surface which are used to attach two key functionalities. A single dendrimer molecule may have at least one hundred arms of each type. One type of arm is used for attachment to targeting molecules (e.g. a capture sequence) to establish target specificity and the other is used for attachment of a label or marker. The molecules that determine the target and labeling specificities of the dendrimer are attached either as oligonucleotides or as oligonucleotide conjugates. Using simple DNA labeling, hybridization, and ligation reactions, the dendrimer probes may be configured to act as a highly labeled, target specific reagent.

To prepare fluorescent labeled dendrimer, the complementary sequences to the capture sequence on the Cy3® RT primer and the Cy5® RT primer are ligated, separately, to the purified dendritic core material as prepared by the previously described methods (see Nilson et al., supra, and U.S. Pat. Nos. '270, '904, and '973, supra.). Thirty nucleotide long oligonucleotides complementary to the outer arms of a four-layer dendrimer having a 5' Cy3® or Cy5® are then synthesized. (Oligos etc., Inc., Wilsonville, OR). The Cy3® and Cy5® oligonucleotides are then hybridized and covalently cross-linked to the outer surface of the corresponding dendrimers, respectively. Excess capture and fluorescent labeled oligonucleotides are then removed through techniques such as size exclusion chromatography.

The concentration of dendrimer is determined by measuring the optical density of the purified material at 260 nm on a UV/Vis spectrometer. The fluorescence is measured at optimal signal/noise wavelengths using a fluorometer (FluoroMax, SPEX Industries). Cy3 is excitable at 542 nm and the emission measured at 570 nm. Cy5 is excitable at 641 nm and the emission at 676 nm.

In previous inventions, the use of dendrimer probes significantly reduces the amount of sample RNA needed to generate an assay while increasing sensitivity due to the dendrimers' superior signal amplification capability. By reducing the amount of RNA required for an assay, the amount of RT primer may be likewise reduced for improved signal generation as discussed below. The reduced RT primer amount also reduces the number of washes needed during assay preparation.

For the assay itself, if desired a strategy can be used (a "two step method") that employs successive hybridization steps where the reverse transcribed cDNA is hybridized overnight to the array in the presence of or after the use of the LNA blocker. Hybridization of the cDNA molecules to target immobilized probes is immediately followed by a washing procedure where the unbound cDNA and LNA blocker is removed from the array. The fluorescently labeled dendrimer molecule (or another molecule capable of binding to the capture sequence incorporated into the cDNA) is added to the washed array and is hybridized to the appropriate target cDNA associated capture sequence during this second hybridization, which typically is performed for 15 - 180 minutes. Excess dendrimer is washed away during a secondary washing procedure and the arrays are scanned as previously discussed.

Alternatively, in accordance with prior inventions the hybridization process can be reduced into a single step ("a one step" process) for increased sensitivity and ease of use, and significant reduction in processing time. The hybridization speed and efficiency is greatly enhanced by first hybridizing the cDNA to the dendrimer probes before hybridizing the cDNA to the microarray. This single-step hybridization process also reduces the number of hybridization buffers to one by eliminating the use of a cDNA hybridization buffer (50% formamide, 10% dextran sulfate, 1X Denhardt's solution, 0.2% N-Lauroyl sarcosine, 250 micrograms/microliter sheared salmon sperm DNA, 2X SSC, 20 mM Tris pH 7.5, and double distilled water). Further details regarding two step and one step processes are provided in PCT Application No. PCT/US01/07477 filed March 8, 2001.

If desired, in further preferred embodiments, temperature cycling can be used to selectively control hybridization between the target nucleic acid and the microarray, and hybridization between the capture reagent and the microarray (preferably cDNA - microarray hybridization and cDNA - dendrimer hybridization, respectively). By using such cycling, hybridization can be carefully controlled such that cDNA initially hybrizides only to the microarray, with subsequent hybridization of cDNA to the dendrimer. This procedure can be used to improve the kinetics of hybridization of each of the two components, i.e. target nucleic acid to probe, and capture reagent to target nucleic acid. Further details regarding use of such temperature cycling are provided in U.S. Provisional Application No. 60/261,231 filed Jan. 13, 2001 (US 2002 09 45 38) and published protocols by the present inventors and by Genisphere, Inc. of Montvale, New Jersey.

The target nucleic acid will generally be DNA that has been reverse transcribed from RNA derived from a naturally occurring source, where the RNA may be selected from the group consisting of total RNA, poly(A)⁺mRNA, amplified RNA and the like. The initial mRNA source may be present in a variety of different samples, where the sample will typically be derived from a physiological source. The physiological source may be derived from a variety of eukaryotic sources, with physiological sources of interest including sources derived from single celled organisms such as yeast and multicellular organisms, including plants and animals, particularly mammals, where the physiological sources from multicellular organisms may be derived from particular organs or tissues of the multicellular organism, or from isolated cells derived therefrom. In obtaining the sample RNAs to be analyzed from the physiological source from which it is derived, the physiological source may be subjected to a number of different processing steps, where such known processing steps may include tissue homogenation, cell isolation and cytoplasmic extraction, nucleic acid extraction and the like. Methods of isolating RNA from cells, tissues, organs or whole organisms are known to those of ordinary skill in the art and are described, for example, in Maniatis et al., Molecular Cloning: A Laboratory Manual, 2nd ed., Cold Spring Harbor Laboratory Press, 1989, and in Ausubel et al., Current Protocols in Molecular Biology, John Wiley & Sons, Inc., 1998.

The sample mRNA is reverse transcribed into a target nucleic acid in the form of a cDNA, by hybridizing an oligo(dT) primer, or RT primer, to the mRNA under conditions sufficient for enzymatic extension of the hybridized primer. The primer will be sufficiently long to provide for efficient hybridization to the mRNA tail, where the region will typically range in length from 10 to 25 nucleotides, usually 10 to 20 nucleotides, and more usually from 12 to 18 nucleotides.

Recognizing that applications typically require the use of sequence specific primers, the standard primers as used in the present invention further include "capture sequence" nucleotide portions. The preferred capture sequences referred to herein are Cy3® RT primer capture sequences (Oligos etc., Inc, Wilsonville, OR) or Cy5® RT primer capture sequence (Oligos etc., Inc, Wilsonville, OR), and are represented below.
Cy3® RT primer capture sequence:
   5' - ggC CTC ACT gCg CgT CTT Ctg TCC CgC C - 3'; and
CY5® RT primer capture sequence:
   5' - CCT gTT gCT CTA TTT CCC gTg Ccg CTC Cgg T - 3'.

For custom primers the above capture sequences should be attached to the 5' end of the corresponding custom oligonucleotide primer. In this manner, the custom primer replaces the standard RT primer. Since the present invention is devised for use with the standard RT primer, some modifications may be required when substituting a custom primer. Such modifications are known to those of ordinary skill in the art and may include adjusting the amount and mixture of primers based on the amount and type of RNA sample used. The primer carries a capture sequence comprised of a specific sequence of nucleotides, as described above. The capture sequence is complementary to the oligonucleotides attached to the arms of dendrimer probes which further carry at least one label. Such complementary oligonucleotides may be acquired from any outside vendor and may also be acquired as labeled moieties. The label may be attached to one or more of the oligonucleotides attached to the arms of the dendrimer probe, either directly or through a linking group, as is known in the art. In the preferred embodiment, the dendrimer probes are labeled by hybridizing and cross-linking Cy3® or Cy5® labeled oligonucleotides to the dendrimer arms. The Cy3® or Cy5® labeled oligonucleotides are complementary to the Cy3® or Cy5® RT primer capture sequences, respectively.

In generating the target nucleic acid sample, the primer is contacted with the mRNA in the presence of a reverse transcriptase enzyme, and other reagents necessary for primer extension under conditions sufficient for inducing first strand cDNA synthesis, where additional reagents include: dNTPs; buffering agents, e.g. Tris.Cl; cationic sources, both monovalent and divalent, e.g. KCl, MgCl₂ ; RNAase inhibitor and sulfhydril reagents, e.g. dithiothreitol; and the like. A variety of enzymes, usually DNA polymerases, possessing reverse transcriptase activity can be used for the first strand cDNA synthesis step. Examples of suitable DNA polymerases include the DNA polymerases derived from organisms selected from the group consisting of a thermophilic bacteria and archaebacteria, retroviruses, yeasts, Neurosporas, Drosophilas, primates and rodents. Suitable DNA polymerases possessing reverse transcriptase activity may be isolated from an organism, obtained commercially or obtained from cells which express high levels of cloned genes encoding the polymerases by methods known to those of skill in the art, where the particular manner of obtaining the polymerase will be chosen based primarily on factors such as convenience, cost, availability and the like. The order in which the reagents are combined may be modified as desired.

In one preferred embodiment, the cDNA synthesis protocol involves combining from about 0.25 to 1 microgram of total RNA or from about 12.5 to 50 nanogram of poly(A)⁺mRNA, with about 0.2 picomole of RT primer (0.2 pmole) and RNase free water for a final volume of about 10 microliters to yield RNA-RT primer mix. The RNA-RT primer mixture is then mixed and microfuged to collect the contents at the bottom of the microfuge tube. The RNA-RT primer mixture is then heated to 80 degrees Celsius for ten minutes and immediately transferred to ice. In a separate microfuge tube on ice, mix together about 4 microliters 5X RT buffer, 1 microliter dNTP mix, 4 microliters RNase free water and 1 microliter of 200 Unit reverse transcriptase enzyme. Gently mix and microfuge briefly to collect the contents at the bottom of the microfuge tube to yield a reaction mixture. Mix the RNA-RT primer mixture with the reaction mixture and then incubate at about 42 degrees Celsius for a period of time sufficient for forming the first strand cDNA primer extension product, which usually takes about 2 hours.

The mixture comprising the cDNA or target nucleic acid subsequent to formation, may be further purified to remove any excess RT primers which may still remain after completion of the reverse transcription process. The excess RT primer would bind to the dendrimer probes resulting in reduced signal strength and intensity and thus reduced assay sensitivity. Although this step is optional, the purification of the cDNA mixture tends to improve the signal strength in the microarray resulting in improved signal generation of the hybridization pattern. The amount of RT primer affects the quality of the assay since excess RT primer can diminish signal strength and resolution. The excess RT primers may be removed from the cDNA mixture by any suitable means including the use of a spin column assembly, QIAquick® PCR Purification Kit (Qiagen, Valencia, CA), and the like. Spin column assemblies are known devices used to separate one or more components from a mixture through centrifugal means.

Preferably, the excess RT primers may be removed via a conventional spin column assembly. The spin column media is composed of a size exclusion resin core which comprises a plurality of resin pores distributed therethrough. The resin pores are sufficiently large to capture the excess RT primer, and permits cDNA to pass into the void volume. To remove excess RT primer, the cDNA containing mixture is placed into a holding tube at one end of the spin column where the spin column and mixture are subjected to high centrifugal force for a period of time. The mixture diffuses through the column and exits at an opposite end into a collecting receptacle. The resulting eluate collected in the receptacle comprises the purified cDNA probe.

In performing the methods of the present invention, a quantity of a labeled dendrimer probe is added to the purified cDNA probe eluate along with a hybridization buffer under temperature conditions which induces hybridization between the dendrimer probe and the target cDNA. In particular, the mixture is incubated at a first pre-hybridization temperature and for a sufficient time to allow the dendrimer probes to attach to the cDNA. The preferred range for the first prehybridization temperature where a formamide-free hybridization buffer is used, is from about 45 to 60 degrees Celsius, and preferably at 55 degrees Celsius. Where a formamide-containing hybridization buffer is used, the preferred range of the pre-hybridization temperature is dependent upon the percent content of formamide where the temperature is reduced by 1 degree Celsius for each 2% formamide present from the standard formamide-free buffer temperature range. The mixture is preferably incubated for about 15 to 20 minutes to allow the cDNA to hybridize with the dendrimer probes to yield a pre-hybridization mix.

The blocking LNA oligonucleotide is preferably added to the mix during the preceding step or just prior to the addition of the dendrimer-cDNA mix to the microarray. Alternatively, the LNA blocker can be prehybridized to the array, i.e. directly added to the array before the application to the array of the target molecules. The blocking LNA oligonucleotide contains both normal and modified LNA base residues (LNA = Locked Nucleic Acid, modified bicyclic monomeric units with a 2'-O- 4'-C methylene bridges) that increase the functional melting point (Tm) of the blocking LNA oligonucliotide (Tm melting points of above 100 degrees Celsius are possible). This allows the blocking LNA oligonucleotide to preferentially and irreversibly hybridize to complementary sequences on the microarray, competing with or replacing RT generated cDNA samples that may have hybridized via these repetitive sequences. (Alternately, the LNA blocker can be used to hybridize to complementary sequences of the target, preferably before application of the target to the array).

Typically, blocking LNA oligonucleotides containing 20-50 normal bases of poly-T or poly-A and 2-20 bases of LNA poly-T or poly-A are selected based on melting point and functional characteristics; the positioning of the LNA residues within the oligonucleotide sequence may alter the functionality of the molecule; therefore, designing the correct oligonucleotide is paramount to appropriate function of the blocking LNA oligonucleotide. Useful and preferred sequence designs have included the following:
Name: Oligo dT36-LNA13
Sequence: 5' - TTt tTt tTT ttt Ttt tTT ttT ttT Ttt tTt ttT ttt - 3'
   where T = LNA residue and t = normal DNA base;
Name: Oligo dA30-LNA7
   5' - aaa aaa aaa aaa aaa aaA aAa AaA aAa AaA -
   where A = LNA residue and a = normal DNA base;

These blocking oligos are designed to block the binding of long extensions of poly-T or poly-A sequences typically found on mRNA and other sequences commonly used for microarray testing. These sequences typically cause false positive results and non-specific signal when hybridization occurs between these sequences and complementary sequences found on the surface of the microarray. Typically, microarray spotted with cDNAs generated from PCR reactions are likely to contain long poly-A and poly-T sequences that are capable of hybridizing the cDNA generated during the reverse transcription reaction described above. (Microarrays containing oligonucleotides as probes are less likely to contain long stretches of monomeric bases, although this is not unknown.)

However, desired blocking LNA oligomers can similarly be used to block any other sequences that could cause non-specific binding interactions, including, but not limited to, repetitive sequences dispersed throughout the genome (human or otherwise). For example, they could be used to block tandemly repeated DNA or interspersed repetitive DNA, whether Short Interspersed Nuclear Elements ("SINES"), Long Interspersed Nuclear Elements ("LINES"), or so forth. Thus, in one embodiment, a blocker could be used to block Alu sequences, or any other sequence currently known or later discovered. In general, any one or more base sequences that could result in non-specific binding to labelled target molecules are identified within the probe or target nucleotides. A LNA blocking reagent can then be provided which has a sequence complementary to those base sequences to bind to them and thereby minimize or eliminate undesirable non-specific probe-target binding interactions.

The pre-hybridization mix is then added to the microarray and incubated at a second hybridization temperature and for a sufficient time to allow the cDNA to bind to the microarray. The preferred range for the second hybridization temperature where a formamide-free hybridization buffer is used; is from about 42 to 60 degrees Celsius. Where a formamide-containing hybridization buffer is used, the preferred range of the pre-hybridization temperature is dependent upon the percent content of formamide where the temperature is reduced by 1 degree Celsius for each 2% formamide present from the standard formamide-free buffer temperature range. Preferably, the pre-hybridization mix and the microarray is incubated at the second temperature overnight in a humidified chamber.

Under such initial conditions, the capture sequence of the cDNA is able to pre-hybridize with the complement attached to the dendrimer probe before the cDNA binds to the gene probe of the microarray. The target cDNA attached to the dendrimer probe, is then contacted with the microarray under conditions sufficient to permit hybridization of the target cDNA to the DNA or gene probe on the microarray. The resulting mixture is incubated overnight for complete hybridization. Suitable hybridization conditions are well known to those of skill in the art and reviewed in Maniatis et al., supra, where conditions may be modulated to achieve a desire specificity in hybridization. It is noted that any suitable hybridization buffers may be used in the present invention. In one preferred form, the hybridization buffer composition may comprise 0.25 M NaPO₄, 4.5% SDS, 1 mM EDTA, and 1X SSC. In another preferred form, the hybridization buffer composition may comprise 40% formamide, 4X SSC, and 1% SDS.

Following the hybridization step, where unhybridized dendrimer probe-cDNA complexes are capable of emitting a signal during the detection step, a washing step is employed where the unhybridized complexes are purged from the microarray, thus leaving behind a visible, discrete pattern of hybridized cDNA-dendrimer probes bound to the microarray. A variety of wash solutions and protocols for their use are known to those of skill in the art and may be used. The specific wash conditions employed will necessarily depend on the specific nature of the signal producing system that is employed, and will be known to those of skill in the art familiar with the particular signal producing system employed.

The resultant hybridization pattern of labeled cDNA fragments may be visualized or detected in a variety of ways, with the particular manner of detection being chosen based on the particular label of the cDNA, where representative detection means include scintillation counting, autoradiography, fluorescence measurement, calorimetric measurement, light emission measurement and the like.

Following hybridization and any washing step(s) and/or subsequent treatments, as described above, the resultant hybridization pattern is detected. In detecting or visualizing the hybridization pattern, the intensity or signal value of the label will be not only be detected but quantified, by which is meant that the signal from each spot of the hybridization will be measured.

Following detection or visualization, the hybridization pattern can be used to determine quantitative and qualitative information about the genetic profile of the labeled target nucleic acid sample that was contacted with the microarray to generate the hybridization pattern, as well as the physiological source from which the labeled target nucleic acid sample was derived. From this data, one can also derive information about the physiological source from which the target nucleic acid sample was derived, such as the types of genes expressed in the tissue or cell which is the physiological source, as well as the levels of expression of each gene, particularly in quantitative terms. Where one uses the subject methods in comparing target nucleic acids from two or more physiological sources, the hybridization patterns may be compared to identify differences between the patterns. Where microarrays in which each of the different probes corresponds to a known gene are employed, any discrepancies can be related to a differential expression of a particular gene in the physiological sources being compared. Thus, the subject methods find use in differential gene expression assays, where one may use the subject methods in the differential expression analysis of: diseased and normal tissue, e.g. neoplastic and normal tissue, different tissue or subtissue types; and the like.

Many other variations of the above procedures can be used consistent with the present invention. For example, instead of utilizing RNA extracted from a sample which is converted to cDNA prior to hybridization, the present invention can be used with the RNA sample directly. In one such embodiment, a suitable capture sequence can be ligated to the RNA using known methods of splicing RNA, such as through enzymatic means. Or, if the RNA includes a specific oligonucleotide that is useful as a capture sequence, a complementary oligonucleotide can be attached to a dendrimer to label the RNA molecule. Further details regarding methods for direct use of RNA without the need for reverse transcription are provided in PCT Application No. PCT/US01/22818 filed July 19, 2001.

Further examples of embodiments of the invention are provided as follows:

### EXAMPLE 1

With reference to Figure 2, a method for detection and assay on a microarray is described below.

### Microarray Preparation

A microarray was prepared as directed by the manufacturer or by customary procedure protocol. The nucleic acid sequences comprising the DNA or gene probes were amplified using known techniques in polymerase chain reaction, then spotted onto glass slides, and processed according to conventional procedures.

### Preparation and Concentration of Target Nucleic Acid Sequences Sample, or cDNA

The target nucleic acid sequences, or cDNA was prepared from total RNA or poly(A)+RNA extracted from a sample of cells. It is noted that for samples containing about 10 to 20 micrograms of total RNA or 500-1000 nanograms of poly(A)⁺ RNA, ethanol precipitation is not required and may be skipped, because the cDNA is sufficiently concentrated to perform the microarray hybridization. In a microfuge tube, 0.25 to 5 micrograms of total RNA or 12.5 to 500 nanograms of poly(A)⁺ RNA was added with 3 microliters of Cy3® or Cy5® RT primer (0.2 pmole) and RNase free water for a total volume of 10FL to yield a RNA-RT primer mixture. The resulting mixture was mixed and microfuged briefly to collect contents in the bottom of the microfuge tube. The collected contents was then heated to 80EC for about ten (10) minutes and immediately transferred to ice. In a separate microfuge tube on ice, 4 microliters of 5X RT buffer, 1 microliter of dNTP mix, 4 microliters RNase free water, and 1microliter of reverse transcriptase enzyme (200 Units) were combined to yield a reaction mixture. The reaction mixture was gently mixed and microfuged briefly to collect contents in the bottom of the microfuge tube. 10 microliters of the RNA-RT primer mixture and 10 microliters of the reaction mixture, was mixed briefly and incubated at 42EC for two hours. The reaction was terminated by adding 3.5 microliters of 0.5 M NaOH/50mM EDTA to the mixture. The mixture was incubated at 65 degrees Celsius for ten (10) minutes to denature the DNA/RNA hybrids and the reaction was neutralized with 5 microliters of 1 M Tris-HCl, pH 7.5. 38.5 microliters of 10 mM Tris, pH 8.0, 1 mM EDTA was then added to the neutralized reaction mixture. (The above steps may be repeated replacing the 3 microliters of Cy3® RT primer (0.2 pmole) with 3 microliters of Cy5® RT primer (0.2 pmole) for preparing dual channel expression assays whereby the prepared Cy3® and Cy5® cDNA mixture are mixed together with 10 microliters of 10 Tris, pH 8.0, 1 mM EDTA, to yield a reaction mixture for processing in the following steps.)

2 microliters of a carrier nucleic acid (10mg/mL linear acrylamide) was added to the neutralized reaction mixture for ethanol precipitation. 175 microliters of 3M ammonium acetate was added to the mixture and then mixed. Then, 625 microliters of 100% ethanol was added to the resulting mixture. The resulting mixture was incubated at -20EC for thirty (30) minutes. The sample was centrifuged at an acceleration rate greater than 10,000 g for fifteen (15) minutes. The supernatant was aspirated and then 330 microliters of 70 % ethanol was added to the supernatant, or cDNA pellet. The cDNA pellet was then centrifuged at an acceleration rate greater than 10,000 g for 5 minutes, and was then removed. The cDNA pellet was dried (i.e., 20-30 minutes at 65 degrees Celsius).

### Hybridization of cDNA/Dendrimer Probe Mixture to Microarray

The DNA hybridization buffer was thawed and resuspended by heating to 65 degrees Celsius for ten (10) minutes. The hybridization buffer comprised of 40% formamide. The buffer was mixed by inversion to ensure that the components were resuspended evenly. The heating and mixing was repeated until all of the material was resuspended. A quantity of competitor DNA was added as required (e.g. COT-1-DNA, and polydA). The cDNA was resuspended in 5.0 microliters of sterile water.

In a first embodiment, single channel analysis, 2.5 microliters of one type of 3DNA® reagent (Genisphere, Inc., Montvale, NJ) (Cy3 or Cy5) was added to the resuspended cDNA along with 12.5 microliters of a DNA hybridization buffer (containing 40% formamide) and 1 microliter the blocking LNA oligonucleotide Oligo dT36-LNA13.

In an alternative embodiment, for dual channel analysis, 2.5 microliters of two types of 3DNA® reagents, Cy3 and Cy5 specifically labeled dendrimers, were added to the resuspended cDNA along with 10 microliters of a DNA hybridization buffer and 1 microliter of the blocking LNA oligonucleotide Oligo dT36-LNA13. In a further embodiment of multiple channel analysis (with three or more channels), 2.5 microliters of three or more types of 3DNA® reagents, Cy3, Cy5, and one or more prepared using another label moiety, were added to the resuspended cDNA along with 10 microliters of a DNA hybridization buffer and 1 microliter of the blocking LNA oligonucleotide Oligo dT36-LNA13.

For larger hybridization buffer volumes, additional DNA hybridization buffer may be added to the required final volume. It is noted that hybridization buffer volumes greater than 35 microliters may also require additional 3DNA® reagents.

The DNA hybridization buffer mixture was incubated at about 50 degrees Celsius for about 15 to 20 minutes to allow for prehybridization of the cDNA to the 3DNA® reagents. The prehybridized mixture was then added to the microarray and then incubated overnight at 55 degrees Celsius. At this stage the cDNA was hybridized to the gene probes.

### Post Hybridization Wash

The microarray was briefly washed to remove any excess dendrimer probes. First, the microarray was washed for 10 minutes at 55 degrees Celsius with 2X SSC buffer, 0.2%SDS. Then the microarray was washed for 10 minutes at room temperature with 2X SSC buffer. Finally the microarray was washed for 10 minutes at room temperature with 0.2X SSC buffer.

### Signal Detection

The microarray was then scanned as directed by the scanner's manufacturer for detecting, analyzing, and assaying the hybridization pattern.

### EXAMPLE 2

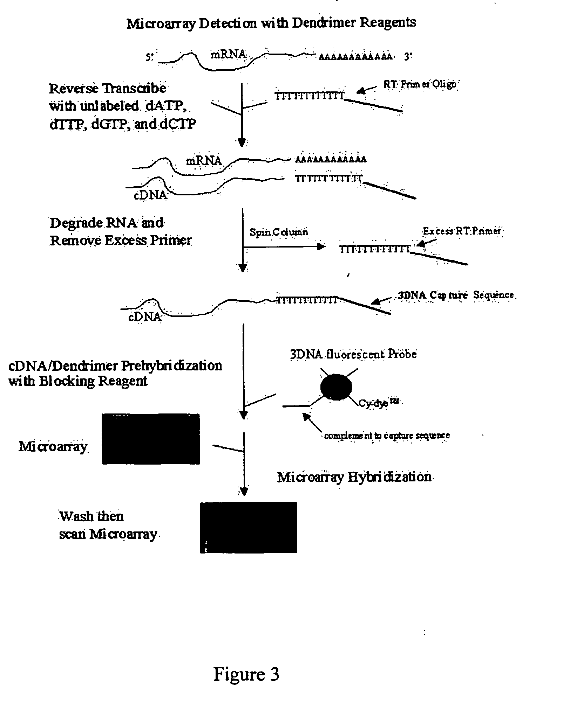

With reference to Figure 3, a method for detection and assay on a microarray is described below. This method includes the use of a spin column assembly for reducing protocol time and number of steps, and for increasing signal strength.

### Microarray Preparation

A microarray was prepared as directed by the manufacturer or by customary protocol procedures. The nucleic acid sequences comprising the DNA or gene probes were amplified using known techniques in polymerase chain reaction, then spotted onto glass slides, and processed according to conventional procedures.

### Preparation and Concentration of Target Nucleic Acid Sequences, or cDNA

The target nucleic acid sequences, or cDNA was prepared from total RNA or poly(A)+RNA extracted from a sample of cells. In a microfuge tube, 0.25 to 5 micrograms of total RNA or 12.5 to 500 ng of poly(A)⁺ RNA was added with 1 microliters of Cy3® or Cy5® RT primer (5 pmole) and RNase free water for a total volume of 10 microliters to yield a RNA-RT primer mixture. The resulting mixture was mixed and microfuged briefly to collect contents in the bottom of the microfuge tube. The collected contents was then heated to 80 degrees Celsius for about ten (10) minutes and immediately transferred to ice. In a separate microfuge tube on ice, 4 microliters of 5X RT buffer, 1 microliter of dNTP mix, 4 microliters RNase free water, and 1 microliter reverse transcriptase enzyme (200 Units) were combined to yield a reaction mixture. The reaction mixture was gently mixed and microfuged briefly to collect contents in the bottom of the microfuge tube. 10 microliters of the RNA-RT primer mixture and 10 microliters of the reaction mixture were mixed together and incubated at 42EC for two hours. The reaction was terminated by adding 3.5 microliters of 0.5 M NaOH/50mM EDTA. The mixture was incubated at 65 degrees Celsius for ten (10) minutes to denature the DNA/RNA hybrids. The reaction was neutralized by the addition of 5 microliters of 1 M Tris-HCl, pH 7.5 to the mixture. 71 microliters of 10 mM Tris, pH 8.0, 1 mM EDTA was added to the neutralized reaction mixture. (The above steps may be repeated replacing the 1 microliter of Cy3® RT primer (5 pmole) with 1 microliter of Cy5® RT primer (5 pmole) for preparing dual channel expression assays whereby the prepared Cy3® and Cy5® cDNA mixture are mixed together with 42 microliters of 10 mM Tris, pH 8.0, 1 mM EDTA, to yield a reaction mixture for processing in the following steps.)

### cDNA Purification: Removal of Excess RT Primer via a SC Spin Column Assembly

The spin column was inverted several times to resuspend the media and to create an even slurry in the column. The top and bottom caps were removed from the spin column. A microfuge tube was obtained and the bottom tip of the microfuge tube, was snipped off or punctured. One end of the spin column was placed into the punctured microfuge tube, then the punctured microfuge tube was placed into a second, intact microfuge tube, or collection tube. The assembled spin column was then placed into a 15 mL centrifuge tube with the microfuge tube end first. The spin column was centrifuged at about 1000 g for about 3.5 minutes after reaching full acceleration. The spin column was checked to ensure that the column was fully drained after centrifugation and that the end of the spin column was above the liquid line in the collection tube. The collection tube contained about 2 to 2.5 mL of clear buffer voided from the spin column. The resin appeared nearly dry in the column barrel, and well packed without distortions or cracks. If the end of the spin column had been immersed in the liquid portion, the spin column would have been discarded and the above steps repeated with a fresh spin column. The spin column was at that point, prepared to remove the excess RT primer in the neutralized reaction mixture.

The drained spin column was removed and a new 1.0 mL collection tube was placed on top of the buffer collection tubes already in the 15 mL centrifuge tube. The voided buffer was discarded. The drained spin column was placed into the new collection tube. 100 microliters of the neutralized reaction mixture containing the cDNA was loaded directly into the center of the spin column media. The spin column assembly was centrifuged at 10,000x g for about 2.5 minutes upon reaching full acceleration. The eluate collected in the new collection tube was then recovered. About 10 percent of the original reaction mixture was recovered. The eluate comprised the cDNA probe.

2 microliters of a carrier nucleic acid (10mg/mL linear acrylamide) was added to the eluate for ethanol precipitation. 250 microliters of 3M ammonium acetate was added to the mixture and mixed. Then, 875 microliters of 100% ethanol was added to the mixture. The resulting mixture was incubated at -20EC for thirty (30) minutes. The sample was centrifuged at an acceleration rate greater than 10,000x g for fifteen (15) minutes. The supernatant was aspirated and 300 microliters of 70 % ethanol was added to the supernatant, or the cDNA pellet. The cDNA pellet was then centrifuged at an acceleration rate greater than 10,000x g for 5 minutes. The supernatant was then removed. The cDNA pellet was dried (i.e. 20-30 minutes at 65 degrees Celsius).

### Hybridization of cDNA/Dendrimer Probe Mixture to Microarray

The DNA hybridization buffer was thawed and resuspended by heating to 65 degrees Celsius and maintained at 65 degrees Celsius for ten (10) minutes. The hybridization buffer comprised of 40% formamide. The buffer was mixed by inversion to ensure that the components were resuspended evenly. The heating and mixing was repeated until all the material was resuspended. A quantity of competitor DNA (e.g. COT-1-DNA, and polydA) may be added, if required. The cDNA was resuspended in 5.0 microliters of sterile water.

In a first embodiment, single channel analysis, 2.5 microliters of one type of 3DNA® reagent (Genisphere, Inc., Montvale, NJ) (Cy3 or Cy5) was added to the resuspended cDNA along with 12.5 microliters of a DNA hybridization buffer (containing 40% formamide) and 1 microliter of the blocking LNA oligonucleotide Oligo dT36-LNA13. In an alternative embodiment, for dual channel analysis, 2.5 microliters of two types of 3DNA® reagents, Cy3 and Cy5 specifically labeled dendrimers, were added to the resuspended cDNA along with 10 microliters of a DNA hybridization buffer and 1 microliter of the blocking LNA oligonucleotide Oligo dT36-LNA13. In a further embodiment of multiple channel analysis (with three or more channels), 2.5 microliters of three or more types of 3DNA® reagents, Cy3, Cy5, and one or more prepared using another label moiety, were added to the resuspended cDNA along with 10 microliters of a DNA hybridization buffer and 1 microliter of the blocking LNA oligonucleotide Oligo dT36-LNA13.

For larger hybridization buffer volumes, additional amounts of the DNA hybridization buffer may be added to reach the required final volume. It is also noted that hybridization buffer volumes greater than 35 microliters may also require additional 3DNA® reagents. The DNA hybridization buffer mixture was incubated at a temperature of about 50 degrees Celsius for about 15 to 20 minutes to allow for the prehybridization of the cDNA to the 3DNA® reagents or dendrimer probes. At this stage, the dendrimer probes of the 3DNA® reagent hybridized with the capture sequence on the cDNA. After 20 minutes, the DNA hybridization buffer was then added to the microarray. The microarray and the DNA hybridization buffer were covered and incubated overnight in a humidified chamber at a temperature of about 55 degrees Celsius. At this stage, the cDNA was hybridized to the gene probes.

### Post Hybridization Wash

The microarray was briefly washed to remove any excess dendrimer probes. First, the microarray was washed for 10 minutes at 55 degrees Celsius with 2X SSC buffer, containing 0.2% SDS. Then, the microarray was washed for 10 minutes at room temperature with 2X SSC buffer. Finally, the microarray was washed for 10 minutes at room temperature with 0.2X SSC buffer.

### Signal Detection

The microarray was then scanned as directed by the scanner's manufacturer for detecting, analyzing, and assaying the hybridization pattern.

### EXAMPLE 3

### An Alternative Method for Detection and Assay on a Microarray

### Microarray Preparation

A microarray was prepared as directed by the manufacturer or by customary protocol procedures. The nucleic acid sequences comprising the DNA or gene probes were amplified using known techniques in polymerase chain reaction, then spotted onto glass slides, and processed according to conventional procedures.

### Preparation and Concentration of Target Nucleic Acid Sequences, or cDNA

The target nucleic acid sequences, or cDNA was prepared from total RNA or poly(A)+RNA extracted from a sample of cells. In a microfuge tube, 0.25 to 10 micrograms of total RNA or 250 to 500 ng of poly(A)⁺ RNA was added with 1 microliters of Cy3® or Cy5® RT primer (5 pmole) and RNase free water for a total volume of 10 microliters to yield a RNA-RT primer mixture. The resulting mixture was mixed and microfuged briefly to collect contents in the bottom of the microfuge tube. The collected contents was then heated to 80 degrees Celsius for about ten (10) minutes and immediately transferred to ice. In a separate microfuge tube on ice, 4 microliters of 5X RT buffer, 1 microliter of dNTP mix, 4 microliter RNase free water, and 1 microliter reverse transcriptase enzyme (200 Units) were combined to yield a reaction mixture. The reaction mixture was gently mixed and microfuged briefly to collect contents in the bottom of the microfuge tube. 10 microliter of the RNA-RT primer mixture and 10 microliters of the reaction mixture was mixed together and incubated at 42 degrees Celsius for two hours. The reaction was terminated by adding 3.5 microliters of 0.5 M NaOH/50mM EDTA. The mixture was incubated at 65 degrees Celsius for ten (10) minutes to denature the DNA/RNA hybrids. The reaction was neutralized by the addition of 5 microliters of 1 M Tris-HCl, pH 7.5 to the mixture. 71 microliters of 10 mM Tris, pH 8.0, 1 mM EDTA was added to the neutralized reaction mixture.

### cDNA Purification: Removal of Excess RT Primer

### via a SC Spin Column Assembly

The spin column was inverted several times to resuspend the media and to create an even slurry in the column. The top and bottom caps were removed from the spin column. A microfuge tube was obtained and the bottom tip of the microfuge tube, was snipped off or punctured. One end of the spin column was placed into the punctured microfuge tube, then the punctured microfuge tube was placed into a second, intact microfuge tube, or collection tube. The assembled spin column was then placed into a 15 mL centrifuge tube with the microfuge tube end first. The spin column was centrifuged at about 1000 g for about 3.5 minutes after reaching full acceleration. The spin column was checked to ensure that the column was fully drained after centrifugation and that the end of the spin column was above the liquid line in the collection tube. The collection tube contained about 2 to 2.5 mL of clear buffer voided from the spin column. The resin appeared nearly dry in the column barrel, and well packed without distortions or cracks. If the end of the spin column had been immersed in the liquid portion, the spin column would have been discarded and the above steps repeated with a fresh spin column. The spin column was prepared to remove the excess RT primer in the neutralized reaction mixture.

The drained spin column was removed and a new 1.0 mL collection tube was placed on top of the buffer collection tubes already in the 15 mL centrifuge tube. The voided buffer was discarded. The drained spin column was placed into the new collection tube. 100 microliters of the neutralized reaction mixture containing the cDNA was loaded directly into the center of the spin column media. The spin column assembly was centrifuged at 10,000x g for about 2.5 minutes upon reaching full acceleration. The eluate collected in the new collection tube was then recovered. About 10 percent of the original reaction mixture was recovered. The eluate comprised the cDNA probe.

### Hybridization of cDNA/Dendrimer Probe Mixture to Microarray

The DNA hybridization buffer was thawed and resuspended by heating to 65EC and maintained at 65 degree Celsius for ten (10) minutes. The hybridization buffer comprised of 40% formamide. The buffer was mixed by inversion to ensure that the components were resuspended evenly. The heating and mixing was repeated until all the material was resuspended. A quantity of competitor DNA (e.g. COT-1-DNA, and polydA) may be added, if required. The cDNA was resuspended in 5.0 microliters of sterile water. In a first embodiment, single channel analysis, 2.5 microliters of one type of 3DNA® reagent (Genisphere, Inc., Montvale, NJ) (Cy3 or Cy5) was added to the resuspended cDNA along with 12.5 microliters of a DNA hybridization buffer (containing 40% formamide) and 1 microliter of the blocking LNA oligonucleotide Oligo dT36-LNA13. In an alternative embodiment, for dual channel analysis, 2.5 microliters of two types of 3DNA® reagents, Cy3 and Cy5 specifically labeled dendrimers, were added to the resuspended cDNA along with 10 microliters of a DNA hybridization buffer and 1 microliter of the blocking LNA oligonucleotide Oligo dT36-LNA13. In a further embodiment of multiple channel analysis (with three or more channels), 2.5 microliters of three or more types of 3DNA® reagents, Cy3, Cy5, and one or more prepared using another label moiety, were added to the resuspended cDNA along with 10 microliters of a DNA hybridization buffer and 1 microliter of the blocking LNA oligonucleotide Oligo dT36-LNA13.

For larger hybridization buffer volumes, additional amounts of the DNA hybridization buffer may be added to reach the required final volume. It is also noted that hybridization buffer volumes greater than 35 microliters may also require additional 3DNA® reagents. The DNA hybridization buffer mixture was incubated at a temperature of about 50 degrees Celsius for about 15 to 20 minutes to allow for the prehybridization of the cDNA to the 3DNA® reagents or dendrimer probes. At this stage, the dendrimer probes of the 3DNA® reagent hybridized with the capture sequence on the cDNA. After 20 minutes, the DNA hybridization buffer was then added to the microarray. The microarray and the DNA hybridization buffer were covered and incubated overnight in a humidified chamber at a temperature of about 55 degrees Celsius. At this stage, the cDNA was hybridized to the gene probes.

### Post Hybridization Wash

The microarray was briefly washed to remove any excess dendrimer probes. First, the microarray was washed for 10 minutes at 55 degrees Celsius C with 2X SSC buffer, containing 0.2%SDS. Then, the microarray was washed for 10 minutes at room temperature with 2X SSC buffer. Finally, the microarray was washed for 10 minutes at room temperature with 0.2X SSC buffer.

### Signal Detection

The microarray was then scanned as directed by the scanner's manufacturer for detecting, analyzing, and assaying the hybridization pattern.

### EXAMPLE 4

### Method for Detection and Assay on a Microarray Using A Detection Kit for cDNA Arrays

### Kit Contents:

- Vial 1: Cy3® 3DNA® Reagent (Genisphere, Montvale, NJ). Use at 2.5 microliters per 20 microliter assay.
- Vial 2: Hybridization buffer- 0.25 M NaPO₄, 4.5% SDS, 1 mM EDTA, and 1X SSC. (Stored at -20 degrees Celsius in the dark.)
- Vial 3: Oligo dT36 LNA-13 Blocking Reagent, 75-125 ng/uL.

### Microarray preparation:

A microarray was prepared as directed by the manufacturer or by customary protocol procedures. The nucleic acid sequences comprising the DNA or gene probes were amplified using known techniques in polymerase chain reaction, then spotted onto glass slides, and processed according to conventional procedures.

### 3DNA® Hybridization:

The hybridization buffer of Vial 2 was thawed and resuspended by heating to 65 degrees Celsius for 10 minutes. The buffer was mixed by inversion to ensure that the components are resuspended evenly. If necessary the heating and mixing was repeated until all the components have resuspended. 2.5 microliters of 3DNA® reagent of Vial 1 was added to 17.5 microliters of hybridization buffer to yield a hybridization mixture. 1 microliter of the blocking LNA oligonucleotide Oligo dT36-LNA13 is also added to the hybridization mixture. The hybridization mixture was added to the microarray. The microarray was covered and incubated at a temperature of from about 37 to 42 degrees Celsius for about 6 hours to overnight in a humidified chamber.

### Post-Hybridization Wash:

The microarray was washed for 10 minutes at 42 degrees Celsius with 2X SSC buffer containing 0.2% SDS. The microarray was then washed for 10 minutes at room temperature with 2X SSC buffer. The microarray was then washed for 10 minutes at room temperature with 0.2X SSC buffer.

### Signal Detection:

The microarray was then scanned as directed by the scanner's manufacturer for detecting, analyzing, and assaying the hybridization pattern.

### EXAMPLE 5

### Method for Detection and Assay on a Microarray Using LNA Blockers and Direct Incorporation of Label

In a microfuge tube 500ng of Oligo(dT) primer was combined with 20µg of total RNA (mouse brain or mouse liver) in a 1.5ml microcentrifuge tube. The volume was adjusted to 21µl with nuclease-free water. The sample was incubated at 75-80°C for 10 minutes, then put on ice immediately for 1-2 minutes. Reverse transcription reaction components were added to the totalRNA and primer as listed below for a total reaction volume of 40µl:
8µl 5X First Strand Buffer (Invitrogen, supplied with enzyme)
4µl 0.1M DTT (Invitrogen, supplied with enzyme)
2µl dNTP mix (10mM dATP, dTTP, dGTP, and 2mM dCTP)
2µl Cy3 or Cy5 modified dCTP (Amersham)
1µl Superase-In (Ambion)
2µl Superscript II RT Enzyme (Invitrogen)

The tube was gently mixed and microfuged to spin down the contents to the bottom of the tube. The reaction was incubated at 42°C for 90 minutes. The reaction stopped by adding 7µl of 0.5M NaOH/50mM EDTA, and incubating the sample for 10 minutes at 65°C. Ten microliters of 1M Tris-HCl was added to neutralize the mixture. The resulting cDNA was purified using the Qiagen QIAquick PCR Purification kit as directed by the manufacturer and was concentrated by ethanol precipitation. The dried cDNA pellet was resuspended in 19.5µl of water. Two microliters of LNA^{™} dT Blocker (25ng/ul), 1µl of Human Cot-1 DNA (1µg/µl), and 22.5µl of 2X SDS based hybridization buffer was added for a total volume of 45µl. The sample was mixed thoroughly, and incubated at 80°C for 15 minutes, with occasional mixing. The sample was pipetted onto a pre-warmed microarray and a 24x60mm glass coverslip was applied to the surface. The array was incubated overnight at 65°C in a humidified hybridization chamber.

The array was removed from hybridization chamber and immersed in 2X SSC, 0.2% . SDS to allow the coverslip to float off the array surface. The arrays were washed as listed below transferring the array from one buffer to the next.
2X SSC, 0.2% SDS at 65°C for 15 minutes.
2X SSC at room temperature for 10 minutes.
0.2X SSC at room temperature for 10 minutes.

The array was transferred to a dry 50mL centrifuge tube and centrifuged for 2 minutes at 800-1000 RPM to dry the slide. The array was removed from the centrifuge tube and scanned to generate the data.

Although the present application discusses several preferred embodiments, further embodiments can be used fully consistent with the invention. For example, as discussed above various embodiments of the invention, LNA can be used as a blocking agent in general in microarray applications, with or without the use of dendrimers. In yet further embodiments, LNA can be used as a blocking agent in non-microarray applications, as well. In other words, consistent with the invention, LNA reagents can be used in any desired application to reduce nonspecific hybridization during the hybridization of cDNA and other nucleic acids to complementary probes.

Furthermore, in the preferred embodiments, Locked Nucleic Acid nucleotides are incorporated into the oligonucleotide in order to confer a greater thermal stability as quantified by Tm and thus improve the usefulness of the reagent as a blocker. Typically, the addition of each LNA nucleotide increases the Tm of the oligonucleotide by approximately 3-5 degrees Celsius depending on the nucleotide substituted or position of placement. Yet, while it is desirable to use an LNA as the nucleotide of choice to increase the Tm, it should be understood that alternative nucleotides can be subtituted in the oligonucleotide to provide similar advantages. For example, other nucleotides wherein the standard nucleotide structure has been modified (i.e. "modified nucleotides") can be utilized. It is preferred that any other such substitute nucleotides or modified nucleotides selected be nucleotides which likewise increase the Tm of the oligonucleotide over the same nucleic acid sequence with "unmodified nucleotides" (i.e. with standard DNA or RNA bases), the Tm elevation preferably being by at least 3-5 degrees Celsius. In addition to any nucleotides currently available in the art, further such nucleotides may become available in the future as organic chemical synthesis continues to advance. Substitution of those nucleotides for the LNA used herein can be used consistent with the present invention for a similar beneficial blocking effect.

The foregoing discussion therefore discloses and describes merely exemplary and preferred embodiments of the present invention. One skilled in the art will readily recognize from such discussion, and from the accompanying drawings, claims, and examples, that various changes, modifications and variations can be made.

## Claims

1. A method comprising:
1) applying a sample to a microarray, wherein the microarray comprises a plurality of features, said features comprising probe nucleotide sequences, wherein said sample comprises target molecules for binding to any of said probe nucleotide sequences on said microarray that are complementary to said target molecules; and,
2) adding an LNA reagent as a blocking reagent to reduce non-specific binding between the target and probe molecules, wherein the LNA reagent is an oligonucleotide containing at least one residue of Locked Nucleic Acid, the Locked Nucleic Acid residues being modified bicyclic monomeric units with a 2'-O - 4'-C methylene bridge,
wherein the LNA reagent includes a sequence which is complementary to a probe or a target sequence that could result in undesirable non-specific binding interactions.

2. The method of claim 1, wherein target molecules comprise a label for producing a detectable signal.

3. The method of claim 1 or 2, wherein said target molecules are cDNA molecules, and further comprising the following steps in the following order:
a) applying said cDNA molecules to said microarray;
b) washing said microarray to remove those of said cDNA molecules which have not hybridized to said microarray; and
c) applying dendrimer molecules to said microarray for hybridization to those of said cDNA molecules which have hybridized to said probe nucleotide sequences.

4. The method of any one of the previous claims 1-3, wherein said LNA reagent is hybridized to said microarray prior to application of said target molecules to said microarray.

5. The method of any one of the previous claims 1-3, wherein said target molecules are hybridized to said LNA reagent, and wherein said target molecules hybridized to said LNA reagent are subsequently applied to said microarray.

6. A method, said method comprising the steps of:
1) contacting a microarray comprising a plurality of features, said features comprising a first set of nucleotide sequences with a mixture comprising:
a) a first component comprising a cDNA reagent obtained from mRNA of a target sample, said cDNA having a capture sequence;
b) a second component comprising a dendrimer having at least one first arm containing a label for producing a detectable signal and at least one second arm having a second nucleotide sequence complementary to said capture sequence;and,
c) a third component comprising an synthetic DNA oligonucleotide containing residues of LNA (Locked Nucleic Acid - modified bicyclic monomeric units with a 2'-O - 4'-C methylene bridge) for use as a blocking reagent on said microarray
wherein the LNA reagent includes a sequence which is complementary to a probe or a target sequence that could result in undesirable non-specific binding interactions.

7. The method of claim 6, further comprising the step of mixing said first component and said second component at a temperature and for a time sufficient to enable said first component to bind to the second component.

8. The method of claim 6 or 7, further comprising the step of incubating said mixture with said microarray to enable said first nucleotide sequence to bind to said first component, wherein such binding results in the feature emitting said detectable signal.

9. The method of any one of the previous claims 6-8, further comprising the step of forming the first component comprising said cDNA reagent by contacting said target sample mRNA with a quantity of an RT primer having the capture sequence, a reverse transcriptase, and nucleotide under conditions sufficient for initiating reverse transcription of said mRNA into said cDNA reagent.

10. The method of claim 9, further comprising the step of purging excess unhybridized RT primer from said first component prior to incubation of said mixture.

11. The method of claim 10, wherein said purging step further comprising the step of passing the first component through a spin column media.

12. The method of any one of the previous claims 6-11, further comprising scanning said microarray for detecting a signal from said label.

13. The method of any one of the previous claims 6-12, further comprising the step of washing said microarray to purge dendrimers unattached to said microarray after the incubation of said microarray and said mixture.

14. A method for detection and assay on a microarray, said method comprising the steps of:
1) incubating a mixture including:
a) a first component comprising a cDNA reagent obtained from mRNA of a target sample, said cDNA having a capture sequence; and
b) a second component comprising a dendrimer having at least one first arm containing a label for producing a detectable signal and at least one second arm having a second nucleotide sequence complementary to the capture sequence,
said mixture being incubated at a first temperature and for a time sufficient to induce the first component to bind to the second component and form a prehybridized cDNA-dendrimer complex;
2) contacting a microarray with said mixture, wherein said microarray comprises a plurality of features, said features comprising a first set of nucleotide sequences;
3) contacting said microarray with a reagent comprising Locked Nucleic Acid (LNA) to reduce non-specific binding of said cDNA reagent to said features of said microarray; and,
4) incubating said microarray and said prehybridized cDNA-dendrimer complex at a second temperature and for a time sufficient to induce said prehybridized cDNA-dendrimer complex to bind any of said set of first nucleotide sequences that are complementary to any sequences of said cDNA reagent, wherein such binding results in said feature emitting said detectable signal such that a hybridization pattern is generated on said microarray,
wherein the LNA reagent includes a sequence which is complementary to a probe or a target sequence that could result in undesirable non-specific binding interactions.

## Patentansprüche

1. Verfahren, umfassend:
1) Auftragen einer Probe auf ein Mikroarray, wobei das Mikroarray eine Mehrzahl von Merkmalen umfasst, wobei die Merkmale Sonden-Nucleotidsequenzen umfassen, wobei die Probe Ziel-Moleküle zum Binden an irgendeine der Sonden-Nucleotidsequenzen auf dem Mikroarray umfasst, die komplementär zu den Ziel-Molekülen sind; und
2) Zugabe eines LNA-Reagens als Blockierungsmittel, um nicht-spezifische Bindung zwischen den Ziel- und Sondenmolekülen zu verringern, wobei das LNA-Reagens ein Oligonucleotid ist, das mindestens einen Locked Nucleic Acid-Rest enthält, wobei die Locked Nucleic Acid-Reste modifizierte bicyclische monomere Einheiten mit einer 2'-O-4'-C-Methylenbrücke sind, wobei das LNA-Reagens eine Sequenz einschließt, die komplementär zu einer Sonden- oder Zielsequenz ist, welche unerwünschte nicht-spezifische Bindungswechselwirkungen zur Folge haben könnte.

2. Verfahren nach Anspruch 1, in dem Ziel-Moleküle einen Marker zur Erzeugung eines nachweisbaren Signals umfassen.

3. Verfahren nach Anspruch 1 oder 2, in dem die Ziel-Moleküle cDNA-Moleküle sind, und das weiter die folgenden Schritte in der folgenden Reihenfolge umfasst:
(a) Auftragen der cDNA-Moleküle auf das Mikroarray;
(b) Waschen des Mikroarrays, um jene cDNA-Moleküle zu entfernen, die nicht mit dem Mikroarray hybridisiert haben; und
(c) Auftragen von Dendrimer-Molekülen auf das Mikroarray zur Hybridisierung jener cDNA-Moleküle, die mit den Sonden-Nucleotidsequenzen hybridisiert haben.

4. Verfahren nach irgendeinem der vorangehenden Ansprüche 1 - 3, in dem das LNA-Reagens vor dem Auftragen der Ziel-Moleküle auf das Mikroarray mit dem Mikroarray hybridisiert wird.

5. Verfahren nach irgendeinem der vorangehenden Ansprüche 1 - 3, in dem die Ziel-Moleküle mit dem LNA-Reagens hybridisiert werden und in dem die Ziel-Moleküle, die an das LNA-Reagens hybridisiert sind, anschließend auf das Mikroarray aufgetragen werden.

6. Verfahren, wobei das Verfahren die Schritte umfasst:
1) In-Kontakt-Bringen eines Mikroarrays, das eine Mehrzahl von Merkmalen umfasst, wobei die Merkmale einen ersten Satz von Nucleotidsequenzen umfassen, mit einer Mischung, welche umfasst:
a) eine erste Komponente, die ein cDNA-Reagens umfasst, das aus mRNA einer Ziel-Probe erhalten wurde, wobei die cDNA eine Einfangsequenz hat;
b) eine zweite Komponente, die ein Dendrimer mit mindestens einem ersten Arm, der einen Marker zur Erzeugung eines nachweisbaren Signals enthält, und mindestens einem zweiten Arm mit einer zweiten Nucleotidsequenz umfasst, die komplementär zu der Einfangsequenz ist; und
c) eine dritte Komponente, die ein synthetisches DNA-Oligonucleotid umfasst, das Reste von LNA (Locked Nucleic Acid - modifizierte bicyclische monomere Einheiten mit einer 2'-O-4'-C-Methylenbrücke) enthält, zur Verwendung als Blockierungsreagens auf dem Mikroarray,
wobei das LNA-Reagens eine Sequenz einschließt, die komplementär zu einer Sonden- oder Zielsequenz ist, welche unerwünschte nicht-spezifische Bindungswechselwirkungen zur Folge haben könnte.

7. Verfahren nach Anspruch 6, weiter umfassend den Schritt des Mischens der ersten Komponente und der zweiten Komponente bei einer Temperatur und über eine ausreichende Zeit, um zu ermöglichen, dass die erste Komponente an die zweite Komponente bindet.

8. Verfahren nach Anspruch 6 oder 7, weiter umfassend den Schritt des Inkubierens der Mischung mit dem Mikroarray, um zu ermöglichen, dass die erste Nucleotidsequenz an die erste Komponente bindet, wobei eine derartige Bindung das Merkmal zum Ergebnis hat, das das nachweisbare Signal emittiert.

9. Verfahren nach irgendeinem der vorangehenden Ansprüche 6 - 8, weiter umfassend den Schritt der Bildung der ersten Komponente, welche das cDNA-Reagens umfasst, durch In-Kontakt-Bringen der Ziel-Proben-mRNA mit einer Menge eines RT-Primers mit der Einfangsequenz, einer reversiblen Transkriptase und von Nucleotid unter ausreichenden Bedingungen, um die reverse Transkription der mRNA in das cDNA-Reagens zu initiieren.

10. Verfahren nach Anspruch 9, weiter umfassend den Schritt der Reinigung von überschüssigem, nicht-hybridisiertem RT-Primer aus der ersten Komponente vor Inkubieren der Mischung.

11. Verfahren nach Anspruch 10, in dem der Reinigungsschritt weiter den Schritt des Durchleitens der ersten Komponente durch ein Spinsäulen-Medium umfasst.

12. Verfahren nach irgendeinem der vorangehenden Ansprüche 6 bis 11, weiter umfassend das Scannen des Mikroarrays zum Nachweisen eines Signals aus dem Marker.

13. Verfahren nach irgendeinem der vorangehenden Ansprüche 6 - 12, weiter umfassend den Schritt des Waschens des Mikroarrays, um Dendrimere abzuspülen, die nach der Inkubation des Mikroarrays und der Mischung nicht an dem Mikroarray angebracht sind.

14. Verfahren für einen Nachweis und Assay auf einem Mikroarray, wobei das Verfahren die Schritte umfasst:
1) Inkubieren einer Mischung, welche einschließt:
a) eine erste Komponente, die ein cDNA-Reagens umfasst, das aus mRNA aus einer Ziel-Probe erhalten wurde, wobei die cDNA eine Einfangsequenz aufweist; und
b) eine zweite Komponente, die ein Dendrimer mit mindestens einem ersten Arm, der einen Marker zur Erzeugung eines nachweisbaren Signals enthält, und mindestens einem zweiten Arm mit einer zweiten Nucleotidsequenz aufweist, welche komplementär zu der Einfangsequenz ist,
wobei die Mischung bei einer ersten Temperatur und über eine ausreichende Zeit inkubiert wird, um zu induzieren, dass die erste Komponente an die zweite Komponente bindet und einen vorhybridisierten cDNA-Dendrimer-Komplex bildet;
2) In-Kontakt-Bringen eines Mikroarrays mit der Mischung, wobei das Mikroarray eine Mehrzahl von Merkmalen umfasst, wobei die Merkmale einen ersten Satz von Nucleotidsequenzen umfassen,
3) In-Kontakt-Bringen des Mikroarrays mit einem Reagens, das Locked Nucleic Acid (LNA) umfasst, um nicht-spezifische Bindung des cDNA-Reagens an die Merkmale des Mikroarrays zu verringern; und
4) Inkubieren des Mikroarrays und des vorhybridisierten cDNA-Dendrimer-Komplexes bei einer zweiten Temperatur und über eine ausreichende Zeit, um zu induzieren, dass der vorhybridisierte cDNA-Dendrimer-Komplex irgendeinen Teil des Satzes von ersten Nucleotidsequenzen bindet, welche komplementär zu irgendwelchen Sequenzen des cDNA-Reagens sind, wobei eine derartige Bindung zur Folge hat, dass das Merkmal das nachweisbare Signal emittiert, so dass ein Hybridisierungsmuster auf dem Mikroarray erzeugt wird,
wobei das LNA-Reagens eine Sequenz einschließt, die komplementär zu einer Proben- oder einer Ziel-Sequenz ist, welche unerwünschte nicht-spezifische Bindungswechselwirkungen zur Folge haben könnte.

## Revendications

1. Procédé comprenant :
1) l'application d'un échantillon à une puce à ADN,
dans lequel la puce à ADN comprend une pluralité de caractéristiques, lesdites caractéristiques comprenant des séquences nucléotidiques de type sonde, ledit échantillon comprenant des molécules cibles pour la liaison à l'une quelconque desdites séquences nucléotidiques sondes sur ladite puce à ADN qui sont complémentaires desdites molécules cibles ; et
2) l'ajout d'un réactif LNA à titre de réactif de blocage pour réduire la liaison non spécifique entre les molécules cibles et sondes, dans lequel le réactif LNA est un oligonucléotide contenant au moins un résidu Acide nucléique verrouillé (LNA), les résidus Acide nucléique verrouillé étant des motifs monomères bicycliques modifiés à pont 2'-O-4'-C méthylène, dans lequel le réactif LNA comprend une séquence qui est complémentaire d'une séquence sonde ou cible qui pourrait induire des interactions de liaison non spécifique indésirables.

2. Procédé selon la revendication 1, dans lequel les molécules cibles comprennent un marqueur pour produire un signal détectable.

3. Procédé selon la revendication 1 ou 2, dans lequel lesdites molécules sondes sont des molécules d'ADNc, et comprenant, en outre, les étapes suivantes, dans l'ordre suivant :
a) application desdites molécules d'ADNc à ladite puce à ADN ;
b) lavage de ladite puce à ADN pour éliminer lesdites molécules d'ADNc qui ne sont pas hybridées à ladite puce à ADN ; et
c) application de molécules dendrimères à ladite puce à ADN pour l'hybridation auxdites molécules d'ADNc qui sont hybridées auxdites séquences nucléotidiques sondes.

4. Procédé selon l'une quelconque des revendications 1 à 3 précédentes, dans lequel le réactif LNA est hybridé à ladite puce à ADN avant l'application desdites molécules cibles à ladite puce à ADN.

5. Procédé selon l'une quelconque des revendications 1 à 3 précédentes, dans lequel lesdites molécules cibles sont hybridées audit réactif LNA, et dans lequel lesdites molécules cibles hybridées audit réactif LNA sont ensuite appliquées à ladite puce à ADN.

6. Procédé, ledit procédé comprenant l'étape de
1) mise en contact d'une puce à ADN comprenant une pluralité de caractéristiques, lesdites caractéristiques comprenant un premier jeu de séquences nucléotidiques, avec un mélange comprenant :
a) un premier composant comprenant un réactif ADNc obtenu à partir de l'ARNm d'un échantillon cible, ledit ADNc contenant une séquence de capture;
b) un deuxième composant comprenant un dendrimère ayant au moins un premier bras contenant un marqueur pour produire un signal détectable et au moins un second bras portant une seconde séquence nucléotidique complémentaire de ladite séquence de capture ; et,
c) un troisième composant comprenant un oligonucléotide de type ADN de synthèse contenant des résidus LNA (Acide nucléique verrouillé - motifs monomères bicycliques modifiés à pont 2'-O-4'-C méthylène), utilisable à titre de réactif de blocage sur ladite puce à ADN,
dans lequel ledit réactif LNA comprend une séquence qui est complémentaire d'une séquence sonde ou cible qui pourrait induire des interactions de liaison non spécifique indésirables.

7. Procédé selon la revendication 6, comprenant, en outre, l'étape de mélange dudit premier composant et dudit deuxième composant à une température et pendant un temps suffisants pour permettre audit premier composant de se lier au deuxième composant.

8. Procédé selon la revendication 6 ou 7, comprenant en outre l'étape d'incubation dudit mélange avec ladite puce à ADN pour permettre la liaison de ladite première séquence nucléotidique audit premier composant, dans lequel cette liaison induit la caractéristique émettant ledit signal détectable.

9. Procédé selon l'une quelconque des revendications 6 à 8, comprenant en outre l'étape de formation du premier composant comprenant ledit réactif ADNc par mise en contact de l'ARNm dudit échantillon cible avec une certaine quantité d'amorce RT contenant la séquence de capture, une transcriptase inverse, et un nucléotide dans des conditions suffisantes pour déclencher la transcription inverse dudit ARNm dans ledit réactif ADNc.

10. Procédé selon la revendication 9, comprenant en outre l'étape de purge de l'amorce RT non hybridée en excédent dudit premier composant avant incubation dudit mélange.

11. Procédé selon la revendication 10, dans lequel ladite étape de purge comprend en outre l'étape du passage du premier composant dans un milieu de colonne spin.

12. Procédé selon l'une quelconque des revendications 6 à 11 précédentes, comprenant en outre le scannage de ladite puce à ADN pour détecter un signal provenant dudit marqueur.

13. Procédé selon l'une quelconque des revendications 6 à 12 précédentes, comprenant en outre l'étape de lavage de ladite puce à ADN pour purger les dendrimères non attachés à ladite puce à ADN après incubation de ladite puce à ADN et dudit mélange.

14. Procédé de détection et de dosage sur puce à ADN, ledit procédé comprenant les étapes suivantes :
1) incubation d'un mélange comprenant :
a) un premier composant comprenant un réactif ADNc obtenu à partir de l'ARNm d'un échantillon cible, ledit ADNc contenant une séquence de capture ; et
b) un second composant comprenant un dendrimère ayant au moins un premier bras contenant un marqueur pour produire un signal détectable et au moins un second bras portant une seconde séquence nucléotidique complémentaire de la séquence de capture,
ledit mélange étant incubé à une première température et pendant un temps suffisants pour induire la liaison du premier composant au second composant et former un complexe ADNc-dendrimère pré-hybridé ;
2) mise en contact d'une puce à ADN avec ledit mélange, ladite puce à ADN comprenant une pluralité de caractéristiques, lesdites caractéristiques comprenant un premier jeu de séquences nucléotidiques ;
3) mise en contact de ladite puce à ADN avec un réactif comprenant un Acide nucléique verrouillé (LNA) pour réduire la liaison non spécifique dudit réactif ADNc auxdites caractéristiques de ladite puce à ADN ; et,
4) incubation de ladite puce à ADN et dudit complexe ADNc-dendrimère pré-hybridé à une seconde température et pendant un temps suffisants pour induire la liaison du complexe ADNc-dendrimère pré-hybridé audit premier jeu de séquences nucléotidiques qui sont complémentaires d'une séquence quelconque dudit réactif ADNc, cette liaison induisant ladite caractéristique émettant ledit signal détectable de façon qu'un profil d'hybridation soit généré sur ladite puce à ADN,
dans lequel le réactif LNA comprend une séquence qui est complémentaire d'une séquence sonde ou cible qui pourrait induite des interactions de liaison non spécifique indésirables.
